# EUROPEAN PATENT APPLICATION

(11) **EP 4 321 618 A1**
(43) Date of publication of application: **14.02.2024**
(21) Application number: 22189465.2
(22) Date of filing: 09.08.2022
(51) Int. Cl.: C12N 9/50, C12N 9/64

(54) **VARIANTS OF TEV PROTEASE AND USES THEREOF**

(71) Applicant: NUMAFERM GmbH, 40225 Düsseldorf (DE); Technische Universität München, 80333 München (DE)
(72) Inventor: SCHWARZ, Christian, 41468 Neuss (DE); ANTES, Iris, 85354 Freising (DE); SIEBER, Volker, 94315 Straubing (DE); WETZEL, Bach-Ngan, 40225 Düsseldorf (DE)
(74) Representative: Maiwald GmbH

(57) **Abstract**

The present invention relates to variants of TEV protease that have - compared to the wildtype enzyme - increased stability and catalytic activity as well as altered substrate specificity. The invention further relates to compositions comprising these variants as well as uses thereof and methods in which these variants are employed.

## Description

### FIELD OF THE INVENTION

The present invention lies in the field of molecular biology, specifically the design and optimization of enzyme variants. Specifically, the present invention is directed to variants of TEV protease that have - compared to the wildtype enzyme - increased stability and catalytic activity as well as altered substrate specificity. The invention further relates to compositions comprising these variants as well as uses thereof and methods in which these variants are employed.

### BACKGROUND OF THE INVENTION

Proteases are an important tool in modern biotechnology and widely used to generate peptides, digest polypeptides, for example for sequencing applications, and cleave undesired amino acid stretches, such as peptide tags used for purification, from the peptide or polypeptide of interest.

To date, one of the most frequently used proteases in such applications is TEV protease, a cysteine protease derived from Tobacco etch virus (TEV) with a molecular weight of 27 kDa. The widespread use of TEV protease is mainly due to its high sequence specificity, since it recognizes a 7 amino acid long consensus motif and cleaves the peptide bond between the penultimate and ultimate amino acid, i.e. between amino acids 6 and 7 of the motif. Further advantages are that it is highly active in mammalian cytosol and requires no cofactors. The high sequence specificity minimizes its activity towards other proteins and allows its use not only *in vitro* methods, but also in intracellular applications.

Despite its many advantages, the use of TEV protease is however hampered by its slow catalysis. Even for its optimal recognition sequence motif, the catalytic activity is significantly lower than that of other proteases, such as trypsin and subtilisin. While there have been some efforts to improve catalytic activity of TEV protease by directed evolution approaches, such as described in international patent publication WO 2021/062063 A1, there is still need for further improved TEV protease variants that may be used in various biotechnological applications and overcome the drawbacks of the wildtype enzyme.

### SUMMARY OF THE INVENTION

The present invention is based on the inventors' surprising finding of variants of TEV protease that exhibit increased catalytic activity and stability and are thus better suited for a variety of biotechnological applications, such as the cleavage of fusion proteins.

In a first aspect, the present invention therefore relates to a polypeptide comprising an amino acid sequence that has at least 80 %, at least 85 %, at least 90 %, at least 95%, at least 97.5%, at least 99% or at least 99.5% sequence identity over its entire length with the amino acid sequence set forth in SEQ ID NO:1 and comprises one or more amino acid substitution(s) selected from the group consisting of: M218F, T22V/I, T30A, D148N/R, Q74L, and S135G, wherein the positions 17, 68, 77, 219 (scaffold), 46, 81 and 151 (catalytic triad) are invariable, wherein the positional numbering is according to SEQ ID NO:1, or a functional fragment thereof.

In various embodiments, the polypeptide further comprises any one or more of the amino acid substitution(s) I138T, S153N and R203G, wherein the positional numbering is according to SEQ ID NO:1.

In various embodiments, the amino acid sequence that has at least 80 %, at least 85 %, at least 90 %, at least 95%, at least 97.5%, at least 99% or at least 99.5% sequence identity over its entire length with the amino acid sequence set forth in SEQ ID NO:1 at its C-Terminus retains the deletion of amino acids 237-242 relative to the TEV protease wildtype sequence set forth in SEQ ID NO:2.

In various embodiments, the functional fragment is at least 202 amino acids in length and comprises amino acid residues corresponding to those at positions 17 to 218 of SEQ ID NO:1.

In various embodiments, the polypeptide comprises
(1) the amino acid substitution M218F and optionally any one or more of T22V/I, T30A, D148N/R, Q74L, and S135G;
(2) the amino acid substitution M218F and optionally any one or more of T22V/I, T30A, D148N/R, Q74L, S135G, I138T, S153N and R203G;
(3) the amino acid substitution M218F and any two, any three, any four, or all five of T22V/I, T30A, D148N/R, Q74L, and S135G;
(4) the amino acid substitution M218F and any two, any three, any four, any five, any six, any seven, or all 8 of T22V/I, T30A, D148N/R, Q74L, S135G, I138T, S153N and R203G;
(5) the amino acid substitutions M218F and T22V/I and optionally any one, any two, any three, or all four of T30A, D148N/R, Q74L, and S135G;
(6) the amino acid substitutions M218F and T22V/I and optionally any one, any two, any three, any four, any five, any six, or all seven of T30A, D148N/R, Q74L, S135G, I138T, S153N and R203G;
(7) the amino acid substitutions M218F and T30A and optionally any one, any two, any three, or all four of T22V/I, D148N/R, Q74L, and S135G;
(8) the amino acid substitutions M218F and T30A and optionally any one, any two, any three, any four, any five, any six, or all seven of T22V/I, D148N/R, Q74L, S135G, I138T, S153N and R203G;
(9) the amino acid substitutions M218F and D148N/R and optionally any one, any two, any three, or all four of T22V/I, T30A, Q74L, and S135G;
(10) the amino acid substitutions M218F and D148N/R and optionally any one, any two, any three, any four, any five, any six, or all seven of T22V/I, T30A, Q74L, S135G, I138T, S153N and R203G;
(11) the amino acid substitutions M218F and Q74L and optionally any one, any two, any three, or all four of T22V/I, T30A, D148N/R, and S135G;
(12) the amino acid substitutions M218F and Q74L and optionally any one, any two, any three, any four, any five, any six, or all seven of T22V/I, T30A, D148N/R, S135G, I138T, S153N and R203G;
(13) the amino acid substitutions M218F and S135Gand optionally any one, any two, any three, or all four of T22V/I, T30A, D148N/R, and Q74L;
(14) the amino acid substitutions M218F and S135G and optionally any one, any two, any three, any four, any five, any six, or all seven of T22V/I, T30A, D148N/R, Q74L, I138T, S153N and R203G;
(15) the amino acid substitutions M218F and I138T and optionally any one, any two, any three, any four, any five, any six, or all seven of T22V/I, T30A, D148N/R, Q74L, S135G, S153N and R203G;
(16) the amino acid substitutions M218F and S153N and optionally any one, any two, any three, any four, any five, any six, or all seven of T22V/I, T30A, D148N/R, Q74L, S135G, I138T and R203G;
(17) the amino acid substitutions M218F and R203G and optionally any one, any two, any three, any four, any five, any six, or all seven of T22V/I, T30A, D148N/R, Q74L, S135G, I138T and S153N;
(18) the amino acid substitutions M218F, S153N and R203G and optionally any one, any two, any three, any four, any five, or all six of T22V/I, T30A, D148N/R, Q74L, S135G and I138T;
(19) the amino acid substitution T22V/I and optionally any one or more of T30A, D148N/R, Q74L, S135G, and M218F;
(20) the amino acid substitution T22V/I and optionally any one or more of T30A, D148N/R, Q74L, S135G, I138T, S153N, R203G and M218F;
(21) the amino acid substitution T22V/I and any two, any three, any four, or all five of T30A, D148N/R, Q74L, S135G, and M218F;
(22) the amino acid substitution T22V/I and any two, any three, any four, any five, any six, any seven, or all eight of T30A, D148N/R, Q74L, S135G, I138T, S153N, R203G, and M218F;
(23) the amino acid substitutions T22V/I and T30A and optionally any one, any two, any three, r all four of M218F, D148N/R, Q74L, and S135G;
(24) the amino acid substitution T22V/I and T30A and optionally any one, any two, any three, any four, any five, any six, or all seven of M218F, D148N/R, Q74L, S135G, I138T, S153N and R203G;
(25) the amino acid substitutions T22V/I and D148N/R and optionally any one, any two, any three, or all four of M218F, T30A, Q74L, and S135G;
(26) the amino acid substitution T22V/I and D148N/R and optionally any one, any two, any three, any four, any five, any six, or all seven of M218F, T30A, Q74L, S135G, I138T, S153N and R203G;
(27) the amino acid substitutions T22V/I and Q74L and optionally any one, any two, any three, or all four of M218F, T30A, D148N/R, and S135G;
(28) the amino acid substitutions T22V/I and Q74L and optionally any one, any two, any three, any four, any five, any six, or all seven of M218F, T30A, D148N/R, S135G, I138T, S153N and R203G;
(29) the amino acid substitutions T22V/I and S135G and optionally any one, any two, any three, or all four of M218F, T30A, D148N/R, and Q74L;
(30) the amino acid substitutions T22V/I and S135G and optionally any one, any two, any three, any four, any five, any six, or all seven of M218F, T30A, D148N/R, Q74L, I138T, S153N and R203G;
(31) the amino acid substitutions T22V/I and I138T and optionally any one, any two, any three, any four, any five, any six, or all seven of M218F, T30A, D148N/R, Q74L, S135G, S153N and R203G;
(32) the amino acid substitutions T22V/I and S153N and optionally any one, any two, any three, any four, any five, any six, or all seven of M218F, T30A, D148N/R, Q74L, S135G, I138T and R203G;
(33) the amino acid substitutions T22V/I and R203G and optionally any one, any two, any three, any four, any five, any six, or all seven of M218F, T30A, D148N/R, Q74L, S135G, I138T and S153N;
(34) the amino acid substitutions T22V/I, S153N and R203G and optionally any one, any two, any three, any four, any five, or all six of M218F, T30A, D148N/R, Q74L, S135G and I138T;
(35) the amino acid substitution T30A and optionally any one or more of T22V/I, D148N/R, Q74L, S135G, and M218F;
(36) the amino acid substitution T30A and optionally any one or more of T22V/I, D148N/R, Q74L, S135G, I138T, S153N, R203G and M218F;
(37) the amino acid substitution T30A and any two, any three, any four, or all five of T22V/I, D148N/R, Q74L, S135G, and M218F;
(38) the amino acid substitution T30A and any two, any three, any four, any five, any six, any seven, or all eight of T22V/I, D148N/R, Q74L, S135G, I138T, S153N, R203G, and M218F;
(39) the amino acid substitutions T30A and D148N/R and optionally any one, any two, any three, or all four of M218F, T22V/I, Q74L, and S135G;
(40) the amino acid substitution T30A and D148N/R and optionally any one, any two, any three, any four, any five, any six, or all seven of M218F, T22V/I, Q74L, S135G, I138T, S153N and R203G;
(41) the amino acid substitutions T30A and Q74L and optionally any one, any two, any three, or all four of M218F, T22V/I, D148N/R, and S135G;
(42) the amino acid substitutions T30A and Q74L and optionally any one, any two, any three, any four, any five, any six, or all seven of M218F, T22V/I, D148N/R, S135G, I138T, S153N and R203G;
(43) the amino acid substitutions T30A and S135G and optionally any one, any two, any three, or all four of M218F, T22V/I, D148N/R, and Q74L;
(44) the amino acid substitutions T30A and S135G and optionally any one, any two, any three, any four, any five, any six, or all seven of M218F, T22V/I, D148N/R, Q74L, I138T, S153N and R203G;
(45) the amino acid substitutions T30A and I138T and optionally any one, any two, any three, any four, any five, any six, or all seven of M218F, T22V/I, D148N/R, Q74L, S135G, S153N and R203G;
(46) the amino acid substitutions T30A and S153N and optionally any one, any two, any three, any four, any five, any six, or all seven of M218F, T22V/I, D148N/R, Q74L, S135G, I138T and R203G;
(47) the amino acid substitutions T30A and R203G and optionally any one, any two, any three, any four, any five, any six, or all seven of M218F, T22V/I, D148N/R, Q74L, S135G, I138T and S153N;
(48) the amino acid substitutions T30A, S153N and R203G and optionally any one, any two, any three, any four, any five, or all six of M218F, T22V/I, D148N/R, Q74L, S135G and I138T;
(49) the amino acid substitution D148N/R and optionally any one or more of T22V/I, T30A, Q74L, S135G, and M218F;
(50) the amino acid substitution D148N/R and optionally any one or more of T22V/I, T30A, Q74L, S135G, I138T, S153N, R203G and M218F;
(51) the amino acid substitution D148N/R and any two, any three, any four, or all five of T22V/I, T30A, Q74L, S135G, and M218F;
(52) the amino acid substitution D148N/R and any two, any three, any four, any five, any six, any seven, or all eight of T22V/I, T30A, Q74L, S135G, I138T, S153N, R203G, and M218F;
(53) the amino acid substitutions D148N/R and Q74L and optionally any one, any two, any three, or all four of M218F, T22V/I, T30A, and S135G;
(54) the amino acid substitutions D148N/R and Q74L and optionally any one, any two, any three, any four, any five, any six, or all seven of M218F, T22V/I, T30A, S135G, I138T, S153N and R203G;
(55) the amino acid substitutions D148N/R and S135G and optionally any one, any two, any three, or all four of M218F, T22V/I, T30A, and Q74L;
(56) the amino acid substitutions D148N/R and S135G and optionally any one, any two, any three, any four, any five, any six, or all seven of M218F, T22V/I, T30A, Q74L, I138T, S153N and R203G;
(57) the amino acid substitutions D148N/R and I138T and optionally any one, any two, any three, any four, any five, any six, or all seven of M218F, T22V/I, T30A, Q74L, S135G, S153N and R203G;
(58) the amino acid substitutions D148N/R and S153N and optionally any one, any two, any three, any four, any five, any six, or all seven of M218F, T22V/I, T30A, Q74L, S135G, I138T and R203G;
(59) the amino acid substitutions D148N/R and R203G and optionally any one, any two, any three, any four, any five, any six, or all seven of M218F, T22V/I, T30A, Q74L, S135G, I138T and S153N;
(60) the amino acid substitutions D148N/R, S153N and R203G and optionally any one, any two, any three, any four, any five, or all six of M218F, T22V/I, T30A, Q74L, S135G and I138T;
(61) the amino acid substitution Q74L and optionally any one or more of T22V/I, T30A, D148N/R, S135G, and M218F;
(62) the amino acid substitution Q74L and optionally any one or more of T22V/I, T30A, D148N/R, S135G, I138T, S153N, R203G and M218F;
(63) the amino acid substitution Q74L and any two, any three, any four, or all five of T22V/I, T30A, D148N/R, S135G, and M218F;
(64) the amino acid substitution Q74L and any two, any three, any four, any five, any six, any seven, or all eight of T22V/I, T30A, D148N/R, S135G, I138T, S153N, R203G, and M218F;
(65) the amino acid substitutions Q74L and S135G and optionally any one, any two, any three, or all four of M218F, T22V/I, T30A, and D148N/R;
(66) the amino acid substitutions Q74L and S135G and optionally any one, any two, any three, any four, any five, any six, or all seven of M218F, T22V/I, T30A, D148N/R, I138T, S153N and R203G;
(67) the amino acid substitutions Q74L and I138T and optionally any one, any two, any three, any four, any five, any six, or all seven of M218F, T22V/I, T30A, D148N/R, S135G, S153N and R203G;
(68) the amino acid substitutions Q74L and S153N and optionally any one, any two, any three, any four, any five, any six, or all seven of M218F, T22V/I, T30A, D148N/R, S135G, I138T and R203G;
(69) the amino acid substitutions Q74L and R203G and optionally any one, any two, any three, any four, any five, any six, or all seven of M218F, T22V/I, T30A, D148N/R, S135G, I138T and S153N;
(70) the amino acid substitutions Q74L, S153N and R203G and optionally any one, any two, any three, any four, any five, or all six of M218F, T22V/I, T30A, D148N/R, S135G and I138T;
(71) the amino acid substitution S135G and optionally any one or more of T22V/I, T30A, D148N/R, Q74L, and M218F;
(72) the amino acid substitution S135G and optionally any one or more of T22V/I, T30A, D148N/R, Q74L, I138T, S153N, R203G and M218F;
(73) the amino acid substitution S135G and any two, any three, any four, or all five of T22V/I, T30A, D148N/R, Q74L, and M218F;
(74) the amino acid substitution S135G and any two, any three, any four, any five, any six, any seven, or all eight of T22V/I, T30A, D148N/R, Q74L, I138T, S153N, R203G, and M218F;
(75) the amino acid substitutions S135G and I138T and optionally any one, any two, any three, any four, any five, any six, or all seven of M218F, T22V/I, T30A, D148N/R, Q74L, S153N and R203G;
(76) the amino acid substitutions S135G and S153N and optionally any one, any two, any three, any four, any five, any six, or all seven of M218F, T22V/I, T30A, D148N/R, Q74L, I138T and R203G;
(77) the amino acid substitutions S135G and R203G and optionally any one, any two, any three, any four, any five, any six, or all seven of M218F, T22V/I, T30A, D148N/R, Q74L, I138T and S153N;
(78) the amino acid substitutions S135G, S153N and R203G and optionally any one, any two, any three, any four, any five, or all six of M218F, T22V/I, T30A, D148N/R, Q74L and I138T;
(79) the amino acid substitutions M218F, T22V/I and T30A and any one, any two, or all three of D148N/R, Q74L, and S135G;
(80) the amino acid substitutions M218F, T22V/I and D148N/R and any one, any two, or all three of T30A, Q74L, and S135G;
(81) the amino acid substitutions M218F, T30A and D148N/R and any one, any two, or all three of T22V/I, Q74L, and S135G;
(82) the amino acid substitutions T22V/I, T30A and D148N/R and any one, any two, or all three of M218F, Q74L, and S135G;
(83) the amino acid substitutions M218F, T22V/I, T30A and D148N/R and any one, or both of Q74L and S135G; or
(84) the amino acid substitutions M218F, T22V/I, T30A and D148N/R and any one, any two, any three, any four, or all five of Q74L, S135G, I138T, S153N, and R203G.

In various embodiments of the polypeptides disclosed herein, T22V/I is T22V or T22V/I is T22I. In various embodiments of the polypeptides disclosed herein D148N/R is D148R or D148N/R is D148N.

The polypeptides of the invention may be isolated polypeptides or purified polypeptides.

In various embodiments, the polypeptide is up to 236 amino acids in length, for example 202 or 219 amino acids in length.

In various embodiments, the polypeptide has protease activity, in particular TEV protease activity.

In various embodiments, the polypeptide comprises or consists of the amino acid sequence set forth in any one of SEQ ID Nos. 4 to 17.

In another aspect, the present invention pertains to a composition comprising the polypeptide of the invention.

In still another aspect, the invention is directed to a nucleic acid molecule encoding the polypeptide of the invention as well as vectors, such as plasmids, comprising a nucleic acid molecule of the invention. The vector may be an expression vector and may comprise additional nucleic acid sequences necessary to facilitate its function in a host cell. The nucleic acid molecule may be an isolated nucleic acid molecule.

Host cells comprising a nucleic acid molecule or a vector according to the invention also form part of the invention. The host cell may be a prokaryotic host cell, for example an *E.coli* cell.

In another aspect, the invention is directed to a method for the production of a polypeptide as described herein, comprising
(1) cultivating the host cell described herein under conditions that allow the expression of the polypeptide; and
(2) isolating the expressed polypeptide from the host cell.

The method may, in various embodiments, further comprise recovering the expressed polypeptide from the host cell and/or the culture medium.

In still another aspect, the invention relates to a method for the cleavage of a substrate polypeptide comprising the amino acid sequence motif set forth in SEQ ID NO:18 (ENLYFQX), comprising contacting the substrate polypeptide with the polypeptide of any one of claims 1 to 8 under conditions that allow the cleavage of the polypeptide; and optionally purifying the cleaved polypeptide.

In a still further aspect, the invention is directed to the use of the polypeptide of the invention for cleavage of a substrate polypeptide comprising the amino acid motif of SEQ ID NO:18.

In various embodiments of the methods or uses of the invention, the substrate polypeptide is a fusion protein, preferably a non-natural fusion protein.

It is understood that all combinations of the above disclosed embodiments are also intended to fall within the scope of the present invention.

### BRIEF DESCRIPTION OF THE DRAWINGS

**Figure 1** shows the rescreening activity results of 20 selected clones with Val peptide. (**A**) Plate 1 (**B**) Plate 2.
**Figure 2** shows the rescreening results of 20 selected clones with both peptides. Dark grey bars: improvement over double mutant (in fold) for Val peptide. Light grey bars: Relative activity to the standard TEV with Gly peptide (SEQ ID NO:19). Dashed line - Standard TEV (SEQ ID NO:1) / DM (SEQ ID NO:3).
**Figure 3** shows the relative activity of some mutants before and after heat shock.
**Figure 4** shows the relative activity of some mutants with modified fluorogenic substrates, where the P-1 position was varied as indicated. 100% was the activity value in RFU of each variant with Gly, so each substrate was compared with variant's Gly activity levels.
**Figure 5** shows the relative activity of some mutants before and after heat shock including P1rev. P1repeat refers to a second expression of P1 in order to repeat the experiment as the same time as P1rev, obtaining the same result as before.

### DETAILED DESCRIPTION OF THE INVENTION

The terms used herein have, unless explicitly stated otherwise, the meanings as commonly understood in the art.

"At least one", as used herein, relates to one or more, in particular 1, 2, 3, 4, 5, 6, 7, 8, 9, 10 or more.

"Isolated" as used herein in relation to a molecule means that said molecule has been at least partially separated from other molecules it naturally associates with or other cellular components. "Isolated" may mean that the molecule has been purified to separate it from other molecules and components, such as other proteins and nucleic acids and cellular debris.

"Nucleic acid" as used herein includes all natural forms of nucleic acids, such as DNA and RNA. Preferably, the nucleic acid molecules of the invention are DNA.

The term "peptide" is used throughout the specification to designate a polymer of amino acid residues connected to each other by peptide bonds. A peptide according to the present invention may have 2-100 amino acid residues. The terms "protein" and "polypeptide" are used interchangeably throughout the specification to designate a polymer of amino acid residues connected to each other by peptide bonds. A protein or polypeptide according to the present invention has preferably 100 or more amino acid residues.

The term "an N-terminal fragment" relates to a peptide or protein sequence which is in comparison to a reference peptide or protein sequence C-terminally truncated, such that a contiguous amino acid polymer starting from the N-terminus of the peptide or protein remains. In some embodiments, such fragments may have a length of at least 30 amino acids, at least 50 amino acids or at least 70 amino acids.

The term "a C-terminal fragment" relates to a peptide or protein sequence which is in comparison to a reference peptide or protein sequence N-terminally truncated, such that a contiguous amino acid polymer starting from the C-terminus of the peptide or protein remains. In some embodiments, such fragments may have a length of at least 30 amino acids, at least 50 amino acids or at least 70 amino acids.

The term "fusion protein" as used herein concerns two or more peptides and proteins which are N- or C-terminally connected to each other, typically by peptide bonds, including via an amino acid/peptide linker sequence. Such fusion proteins may be encoded by two or more nucleic acid sequences which are operably fused to each other. In certain embodiments, a fusion protein refers to at least one peptide or protein of interest C-terminally or N-terminally fused to the TEV variant amino acid sequence according to the invention, optionally via a linker sequence.

"Stability", as used herein in relation to the polypeptides of the invention, primarily relates to resistance to (proteolytic) degradation and denaturation, which is a commonly encountered issue.

Generally, the skilled person understands that for putting the present invention into practice any nucleotide sequence described herein may comprise an additional start and/or stop codon or that a start and/or stop codon included in any of the sequences described herein may be deleted, depending on the nucleic acid construct used. The skilled person will base this decision, e.g., on whether a nucleic acid sequence comprised in the nucleic acid molecule of the present invention is to be translated and/or is to be translated as a fusion protein. In various embodiments, the polypeptides of the invention additionally comprise the amino acid M on the N-terminus of the polypeptide.

The present invention is based on the inventors finding that certain variants of TEV protease provide for an increased activity and altered substrate specificity as well as increased stability relative to TEV wildtype (SEQ ID NO:2) and known TEV variants (SEQ ID NO:1).

Thus, in a first aspect, the present invention relates to a(n) (isolated) polypeptide comprising an amino acid sequence that has at least 80 %, at least 85 %, at least 90 %, at least 95%, at least 97.5%, at least 99% or at least 99.5% sequence identity over its entire length with the amino acid sequence set forth in SEQ ID NO:1 and comprises one or more amino acid substitution(s) selected from the group consisting of: M218F, T22V/I, T30A, D148N/R, Q74L, and S135G, wherein the positions 46, 81 and 151 and, optionally, 17, 68, 77, and 219 are invariable, wherein the positional numbering is according to SEQ ID NO:1, or a functional fragment thereof. In various embodiments, the polypeptide can additionally comprise one, two or all three of the substitutions: I138T, S153N and R203G. In various embodiments, it is preferred that the polypeptides of the invention further comprise at least the R203G substitution. In such embodiments, the R203G substitution may then also be invariable.

The polypeptide does not include the amino acid sequence as set forth in SEQ ID NO:1 or SEQ ID NO:2, but comprises a variant thereof that comprises at least one substitution as defined above.

The amino acid sequence of the polypeptide of the invention has, over its entire length, at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95%, at least 96 %, at least 97 %, at least 97.5%, at least 98 %, at least 98.5 %, at least 99% or 99.5% sequence identity with the corresponding part of the amino acid sequence set forth in SEQ ID NO:1. In various embodiments, the amino acid sequence is of the same length as the sequence set forth in SEQ ID NO:1. In other embodiments, it is a shortened fragment thereof that may be obtainable by deletions/truncations. Such truncated versions are also referred to herein as functional fragments and are further defined below.

In various embodiments, the sequence of the amino acid sequence with the exception of the above-indicated positions that may be substituted or are invariable, i.e. the remainder of the amino acid sequence that is not substituted or invariable, is essentially identical to the sequence set forth in SEQ ID NO:1, i.e. has sequence identities of at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95%, at least 96 %, at least 97 %, at least 97.5%, at least 98 %, at least 98.5 %, at least 99%, at least 99.5% or 100% with the sequence of SEQ ID NO:1.

Determination of the sequence identity of nucleic acid or amino acid sequences can be done by a sequence alignment based on well-established and commonly used BLAST algorithms (See, e.g. Altschul, S.F., Gish, W., Miller, W., Myers, E.W. & Lipman, D.J. (1990) "Basic local alignment search tool." J. Mol. Biol. 215:403-410, and Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402). Such an alignment is based on aligning similar nucleotide or amino acid sequences stretches with each other. Another algorithm known in the art fo said purpose is the FASTA algorithm. Alignments, in particular multiple sequence comparisons, are typically done by using computer programs. Commonly used are the Clustal series (See, e.g., Chenna et al. (2003): Multiple sequence alignment with the Clustal series of programs. Nucleic Acid Research 31, 3497-3500), T-Coffee (See, e.g., Notredame et al. (2000): T-Coffee: A novel method for multiple sequence alignments. J. Mol. Biol. 302, 205-217) or programs based on these known programs or algorithms.

Also possible are sequence alignments using the computer program Vector NTI^{®} Suite 10.3 (Invitrogen Corporation, 1600 Faraday Avenue, Carlsbad, CA, USA) with the set standard parameters, with the AlignX module for sequence comparisons being based on the ClustalW. If not indicated otherwise, the sequence identity is determined using the BLAST algorithm.

Such a comparison also allows determination of the similarity of the compared sequences. Said similarity is typically expressed in percent identify, i.e. the portion of identical nucleotides/amino acids at the same or corresponding (in an alignment) sequence positions relative to the total number of the aligned nucleotides/amino acids. For example, if in an alignment 90 amino acids of a 100 aa long query sequence are identical to the amino acids in corresponding positions of a template sequence, the sequence identity is 90%. The broader term "homology" additionally considers conserved amino acid substitutions, i.e. amino acids that are similar in regard to their chemical properties, since those typically have similar chemical properties in a protein. Accordingly, such homology can be expressed in percent homology. If not indicated otherwise, sequence identity and sequence homology relate to the entire length of the aligned sequence.

In the context of the present invention, the feature that an amino acid position corresponds to a numerically defined position in SEQ ID NO:1 means that the respective position correlates to the numerically defined position in SEQ ID NO:1 in an alignment obtained as described above.

"Amino acid substitution", as used herein, relates to modification of the sequence such that the amino acid residue occurring at the corresponding position in SEQ ID NO:1 is replaced by another amino acid residue. The amino acid residue for substitution is typically selected from the 20 proteinogenic amino acids G, A, V, L, I, F, C, M, P, R, K, H, N, Q, D, E, S, T, W, and Y. Accordingly, if a position in SEQ ID NO:1 is occupied by any one of these 20 amino acid residues, its substitution means that it is replaced by any one of the other 19 amino acids listed above. A "deletion" at one or more positions means that the residues adjacent to the deletion site are directly connected by a peptide bond.

The amino acids are typically referred to herein in the one-letter code. Furthermore, the nomenclature is used in line with the accepted meaning in the art, such that the starting amino acid is given first in one-letter code, followed by the positional number and then the target amino acid. If there are multiple options for the target amino acid, the individual options are separated by "/". The indication "M218F" thus means that the methionine residue in position 218 or the position corresponding to position 218 is replaced by phenylalanine. The term "T22V/I" thus means that threonine in position 22 or a position corresponding to position 22 is replaced by either valine or isoleucine.

The amino acids in the positions that correspond to positions 46, 81 and 151 (of SEQ ID NO:1) are invariable, as these form the catalytic triad of the enzyme and are thus crucial for its proteolytic activity. These amino acids are typically 46H, 81D and 151C and are generally conserved in the polypeptides of the invention.

The polypeptides of the invention furthermore comprise, relative to TEV wildtype, substitutions in the positions that correspond to positions 17, 68, 77, 219 in SEQ ID NO:1. These are known substitutions that have beneficial effects on solubility and other enzymatic properties and are thus typically retained and invariable in the polypeptides of the invention. These substitutions that are to be retained and already reflected in the amino acid sequence of SEQ ID NO:1 are 17S, 68D, 77V and 219N. Relative to TEV wildtype, these substitutions are T17S, N68D, I77V and S219N.

In various embodiments of the invention, the polypeptides of the invention thus have the invariable amino acids 46H, 81D, 151C, 17S, 68D, 77V and 219N, with the positional numbering according to SEQ ID NO:1, and additionally comprise one or more of the amino acid substitution(s) selected from the group consisting of: M218F, T22V/I, T30A, D148N/R, Q74L, and S135G.

The amino acid sequence of the polypeptide of the present invention is, in various embodiments, up to 242 amino acids in length, preferably 202 to 236 or 202 to 219 amino acids in length. It is preferred that the polypeptide comprises the region that corresponds to amino acids 17 to 218 of SEQ ID NO:1, more preferably also any one or more of the amino acids that correspond to those at positions 219 to 236 and/or 1 to 16. The polypeptide of the invention may be even shorter and comprise only up to the amino acid at position 219, i.e. is a Δ220-242 TEV protease. It is further preferred that it also comprises the amino acids at position(s) 16, 15-16, 14-16, 13-16, 12-16, 11-16, 10-16, 9-16, 8-16, 7-16, 6-16, 5-16, 4-16, 3-16, 2-16 or 1-16, using the positional numbering of SEQ ID NO:1.

In various embodiments, the polypeptide of the invention is a Δ237-242 TEV protease, i.e. lacks relative to the TEV protease wildtype sequence (SEQ ID NO:2), the amino acids in positions 237-242, i.e. the C-terminus. Said deletion is already reflected in SEQ ID NO:1.

While the amino acid sequence may correspond to a continuous amino acid stretch of the amino acid sequence set forth in SEQ ID NO:1 having the indicated length, it is similarly possible that the amino acid sequence corresponds to discontinuous stretches of the amino acid sequence set forth in SEQ ID NO:1, for example if it corresponds to stretches of SEQ ID NO:1 with certain amino acids or amino acid sequences being deleted therefrom. The polypeptide may thus be derived from the amino acid sequence set forth in SEQ ID NO:1 by any one or more of an N-terminal truncation, a C-terminal truncation or a deletion of one or more amino acids, in particular as described above. Any such shortened variants of the amino acid sequence of SEQ ID NO:1 are covered by the term "fragment", as used herein. The term "functional fragment" additionally implies that the respective polypeptide retains its catalytic activity. It is preferred that the fragments of the invention have at least the catalytic activity of the wildtype TEV protease as set forth in SEQ ID NO:2 or, preferably, of the TEV protease variant having the amino acid sequence of SEQ ID NO:1.

In various embodiments, the polypeptides of the invention comprise one, two, three or more of the indicated amino acid substitutions. In various embodiments, the polypeptides comprise
(1) the amino acid substitution M218F and optionally any one or more of T22V/I, T30A, D148N/R, Q74L, and S135G;
(2) the amino acid substitution M218F and optionally any one or more of T22V/I, T30A, D148N/R, Q74L, S135G, I138T, S153N and R203G;
(3) the amino acid substitution M218F and any two, any three, any four, or all five of T22V/I, T30A, D148N/R, Q74L, and S135G;
(4) the amino acid substitution M218F and any two, any three, any four, any five, any six, any seven, or all 8 of T22V/I, T30A, D148N/R, Q74L, S135G, I138T, S153N and R203G;
(5) the amino acid substitutions M218F and T22V/I and optionally any one, any two, any three, or all four of T30A, D148N/R, Q74L, and S135G;
(6) the amino acid substitutions M218F and T22V/I and optionally any one, any two, any three, any four, any five, any six, or all seven of T30A, D148N/R, Q74L, S135G, I138T, S153N and R203G;
(7) the amino acid substitutions M218F and T30A and optionally any one, any two, any three, or all four of T22V/I, D148N/R, Q74L, and S135G;
(8) the amino acid substitutions M218F and T30A and optionally any one, any two, any three, any four, any five, any six, or all seven of T22V/I, D148N/R, Q74L, S135G, I138T, S153N and R203G;
(9) the amino acid substitutions M218F and D148N/R and optionally any one, any two, any three, or all four of T22V/I, T30A, Q74L, and S135G;
(10) the amino acid substitutions M218F and D148N/R and optionally any one, any two, any three, any four, any five, any six, or all seven of T22V/I, T30A, Q74L, S135G, I138T, S153N and R203G;
(11) the amino acid substitutions M218F and Q74L and optionally any one, any two, any three, or all four of T22V/I, T30A, D148N/R, and S135G;
(12) the amino acid substitutions M218F and Q74L and optionally any one, any two, any three, any four, any five, any six, or all seven of T22V/I, T30A, D148N/R, S135G, I138T, S153N and R203G;
(13) the amino acid substitutions M218F and S135Gand optionally any one, any two, any three, or all four of T22V/I, T30A, D148N/R, and Q74L;
(14) the amino acid substitutions M218F and S135G and optionally any one, any two, any three, any four, any five, any six, or all seven of T22V/I, T30A, D148N/R, Q74L, I138T, S153N and R203G;
(15) the amino acid substitutions M218F and I138T and optionally any one, any two, any three, any four, any five, any six, or all seven of T22V/I, T30A, D148N/R, Q74L, S135G, S153N and R203G;
(16) the amino acid substitutions M218F and S153N and optionally any one, any two, any three, any four, any five, any six, or all seven of T22V/I, T30A, D148N/R, Q74L, S135G, I138T and R203G;
(17) the amino acid substitutions M218F and R203G and optionally any one, any two, any three, any four, any five, any six, or all seven of T22V/I, T30A, D148N/R, Q74L, S135G, I138T and S153N;
(18) the amino acid substitutions M218F, S153N and R203G and optionally any one, any two, any three, any four, any five, or all six of T22V/I, T30A, D148N/R, Q74L, S135G and I138T.

In various embodiments, the polypeptides of the invention comprise
(19) the amino acid substitution T22V/I and optionally any one or more of T30A, D148N/R, Q74L, S135G, and M218F;
(20) the amino acid substitution T22V/I and optionally any one or more of T30A, D148N/R, Q74L, S135G, I138T, S153N, R203G and M218F;
(21) the amino acid substitution T22V/I and any two, any three, any four, or all five of T30A, D148N/R, Q74L, S135G, and M218F;
(22) the amino acid substitution T22V/I and any two, any three, any four, any five, any six, any seven, or all eight of T30A, D148N/R, Q74L, S135G, I138T, S153N, R203G, and M218F;
(23) the amino acid substitutions T22V/I and T30A and optionally any one, any two, any three, r all four of M218F, D148N/R, Q74L, and S135G;
(24) the amino acid substitution T22V/I and T30A and optionally any one, any two, any three, any four, any five, any six, or all seven of M218F, D148N/R, Q74L, S135G, I138T, S153N and R203G;
(25) the amino acid substitutions T22V/I and D148N/R and optionally any one, any two, any three, or all four of M218F, T30A, Q74L, and S135G;
(26) the amino acid substitution T22V/I and D148N/R and optionally any one, any two, any three, any four, any five, any six, or all seven of M218F, T30A, Q74L, S135G, I138T, S153N and R203G;
(27) the amino acid substitutions T22V/I and Q74L and optionally any one, any two, any three, or all four of M218F, T30A, D148N/R, and S135G;
(28) the amino acid substitutions T22V/I and Q74L and optionally any one, any two, any three, any four, any five, any six, or all seven of M218F, T30A, D148N/R, S135G, I138T, S153N and R203G;
(29) the amino acid substitutions T22V/I and S135G and optionally any one, any two, any three, or all four of M218F, T30A, D148N/R, and Q74L;
(30) the amino acid substitutions T22V/I and S135G and optionally any one, any two, any three, any four, any five, any six, or all seven of M218F, T30A, D148N/R, Q74L, I138T, S153N and R203G;
(31) the amino acid substitutions T22V/I and I138T and optionally any one, any two, any three, any four, any five, any six, or all seven of M218F, T30A, D148N/R, Q74L, S135G, S153N and R203G;
(32) the amino acid substitutions T22V/I and S153N and optionally any one, any two, any three, any four, any five, any six, or all seven of M218F, T30A, D148N/R, Q74L, S135G, I138T and R203G;
(33) the amino acid substitutions T22V/I and R203G and optionally any one, any two, any three, any four, any five, any six, or all seven of M218F, T30A, D148N/R, Q74L, S135G, I138T and S153N;
(34) the amino acid substitutions T22V/I, S153N and R203G and optionally any one, any two, any three, any four, any five, or all six of M218F, T30A, D148N/R, Q74L, S135G and I138T.

In various embodiments, the polypeptides of the invention comprise
(35) the amino acid substitution T30A and optionally any one or more of T22V/I, D148N/R, Q74L, S135G, and M218F;
(36) the amino acid substitution T30A and optionally any one or more of T22V/I, D148N/R, Q74L, S135G, I138T, S153N, R203G and M218F;
(37) the amino acid substitution T30A and any two, any three, any four, or all five of T22V/I, D148N/R, Q74L, S135G, and M218F;
(38) the amino acid substitution T30A and any two, any three, any four, any five, any six, any seven, or all eight of T22V/I, D148N/R, Q74L, S135G, I138T, S153N, R203G, and M218F;
(39) the amino acid substitutions T30A and D148N/R and optionally any one, any two, any three, or all four of M218F, T22V/I, Q74L, and S135G;
(40) the amino acid substitution T30A and D148N/R and optionally any one, any two, any three, any four, any five, any six, or all seven of M218F, T22V/I, Q74L, S135G, I138T, S153N and R203G;
(41) the amino acid substitutions T30A and Q74L and optionally any one, any two, any three, or all four of M218F, T22V/I, D148N/R, and S135G;(42) the amino acid substitutions T30A and Q74L and optionally any one, any two, any three, any four, any five, any six, or all seven of M218F, T22V/I, D148N/R, S135G, I138T, S153N and R203G;
(43) the amino acid substitutions T30A and S135G and optionally any one, any two, any three, or all four of M218F, T22V/I, D148N/R, and Q74L;
(44) the amino acid substitutions T30A and S135G and optionally any one, any two, any three, any four, any five, any six, or all seven of M218F, T22V/I, D148N/R, Q74L, I138T, S153N and R203G;
(45) the amino acid substitutions T30A and I138T and optionally any one, any two, any three, any four, any five, any six, or all seven of M218F, T22V/I, D148N/R, Q74L, S135G, S153N and R203G;
(46) the amino acid substitutions T30A and S153N and optionally any one, any two, any three, any four, any five, any six, or all seven of M218F, T22V/I, D148N/R, Q74L, S135G, I138T and R203G;
(47) the amino acid substitutions T30A and R203G and optionally any one, any two, any three, any four, any five, any six, or all seven of M218F, T22V/I, D148N/R, Q74L, S135G, I138T and S153N;
(48) the amino acid substitutions T30A, S153N and R203G and optionally any one, any two, any three, any four, any five, or all six of M218F, T22V/I, D148N/R, Q74L, S135G and I138T.

In various embodiments of the invention, the polypeptide comprises
(49) the amino acid substitution D148N/R and optionally any one or more of T22V/I, T30A, Q74L, S135G, and M218F;
(50) the amino acid substitution D148N/R and optionally any one or more of T22V/I, T30A, Q74L, S135G, I138T, S153N, R203G and M218F;
(51) the amino acid substitution D148N/R and any two, any three, any four, or all five of T22V/I, T30A, Q74L, S135G, and M218F;
(52) the amino acid substitution D148N/R and any two, any three, any four, any five, any six, any seven, or all eight of T22V/I, T30A, Q74L, S135G, I138T, S153N, R203G, and M218F;
(53) the amino acid substitutions D148N/R and Q74L and optionally any one, any two, any three, or all four of M218F, T22V/I, T30A, and S135G;
(54) the amino acid substitutions D148N/R and Q74L and optionally any one, any two, any three, any four, any five, any six, or all seven of M218F, T22V/I, T30A, S135G, I138T, S153N and R203G;
(55) the amino acid substitutions D148N/R and S135G and optionally any one, any two, any three, or all four of M218F, T22V/I, T30A, and Q74L;
(56) the amino acid substitutions D148N/R and S135G and optionally any one, any two, any three, any four, any five, any six, or all seven of M218F, T22V/I, T30A, Q74L, I138T, S153N and R203G;
(57) the amino acid substitutions D148N/R and I138T and optionally any one, any two, any three, any four, any five, any six, or all seven of M218F, T22V/I, T30A, Q74L, S135G, S153N and R203G;
(58) the amino acid substitutions D148N/R and S153N and optionally any one, any two, any three, any four, any five, any six, or all seven of M218F, T22V/I, T30A, Q74L, S135G, I138T and R203G;
(59) the amino acid substitutions D148N/R and R203G and optionally any one, any two, any three, any four, any five, any six, or all seven of M218F, T22V/I, T30A, Q74L, S135G, I138T and S153N;
(60) the amino acid substitutions D148N/R, S153N and R203G and optionally any one, any two, any three, any four, any five, or all six of M218F, T22V/I, T30A, Q74L, S135G and I138T.

In various embodiments, the polypeptide of the invention comprises
(61) the amino acid substitution Q74L and optionally any one or more of T22V/I, T30A, D148N/R, S135G, and M218F;
(62) the amino acid substitution Q74L and optionally any one or more of T22V/I, T30A, D148N/R, S135G, I138T, S153N, R203G and M218F;
(63) the amino acid substitution Q74L and any two, any three, any four, or all five of T22V/I, T30A, D148N/R, S135G, and M218F;
(64) the amino acid substitution Q74L and any two, any three, any four, any five, any six, any seven, or all eight of T22V/I, T30A, D148N/R, S135G, I138T, S153N, R203G, and M218F;
(65) the amino acid substitutions Q74L and S135G and optionally any one, any two, any three, or all four of M218F, T22V/I, T30A, and D148N/R;
(66) the amino acid substitutions Q74L and S135G and optionally any one, any two, any three, any four, any five, any six, or all seven of M218F, T22V/I, T30A, D148N/R, I138T, S153N and R203G;
(67) the amino acid substitutions Q74L and I138T and optionally any one, any two, any three, any four, any five, any six, or all seven of M218F, T22V/I, T30A, D148N/R, S135G, S153N and R203G;
(68) the amino acid substitutions Q74L and S153N and optionally any one, any two, any three, any four, any five, any six, or all seven of M218F, T22V/I, T30A, D148N/R, S135G, I138T and R203G;
(69) the amino acid substitutions Q74L and R203G and optionally any one, any two, any three, any four, any five, any six, or all seven of M218F, T22V/I, T30A, D148N/R, S135G, I138T and S153N;
(70) the amino acid substitutions Q74L, S153N and R203G and optionally any one, any two, any three, any four, any five, or all six of M218F, T22V/I, T30A, D148N/R, S135G and I138T.

In various embodiments, the polypeptide of the invention comprises
(71) the amino acid substitution S135G and optionally any one or more of T22V/I, T30A, D148N/R, Q74L, and M218F;
(72) the amino acid substitution S135G and optionally any one or more of T22V/I, T30A, D148N/R, Q74L, I138T, S153N, R203G and M218F;
(73) the amino acid substitution S135G and any two, any three, any four, or all five of T22V/I, T30A, D148N/R, Q74L, and M218F;
(74) the amino acid substitution S135G and any two, any three, any four, any five, any six, any seven, or all eight of T22V/I, T30A, D148N/R, Q74L, I138T, S153N, R203G, and M218F;
(75) the amino acid substitutions S135G and I138T and optionally any one, any two, any three, any four, any five, any six, or all seven of M218F, T22V/I, T30A, D148N/R, Q74L, S153N and R203G;
(76) the amino acid substitutions S135G and S153N and optionally any one, any two, any three, any four, any five, any six, or all seven of M218F, T22V/I, T30A, D148N/R, Q74L, I138T and R203G;
(77) the amino acid substitutions S135G and R203G and optionally any one, any two, any three, any four, any five, any six, or all seven of M218F, T22V/I, T30A, D148N/R, Q74L, I138T and S153N;
(78) the amino acid substitutions S135G, S153N and R203G and optionally any one, any two, any three, any four, any five, or all six of M218F, T22V/I, T30A, D148N/R, Q74L and I138T.

In various embodiments, the polypeptide comprises at least three of the indicated substitutions. In some embodiments, such polypeptides comprise
(79) the amino acid substitutions M218F, T22V/I and T30A and any one, any two, or all three of D148N/R, Q74L, and S135G;
(80) the amino acid substitutions M218F, T22V/I and D148N/R and any one, any two, or all three of T30A, Q74L, and S135G;
(81) the amino acid substitutions M218F, T30A and D148N/R and any one, any two, or all three of T22V/I, Q74L, and S135G;
(82) the amino acid substitutions T22V/I, T30A and D148N/R and any one, any two, or all three of M218F, Q74L, and S135G;
(83) the amino acid substitutions M218F, T22V/I, T30A and D148N/R and any one, or both of Q74L and S135G;
(84) the amino acid substitutions M218F, T22V/I, T30A and D148N/R and any one, any two, any three, any four, or all five of Q74L, S135G, I138T, S153N, and R203G.

In all embodiments disclosed herein, T22V/I may be T22V. Alternatively, in all embodiments disclosed herein T22V/I may be T22I. In some embodiments, T22V may be preferred over T22I.

In all embodiments disclosed herein, D148N/R may be D148N. Alternatively, in all embodiments disclosed herein D148N/R may be D148R.

In various embodiments, the polypeptide comprises the amino acid sequence set forth in any one of SEQ ID Nos. 4-17. In further embodiments, also encompassed are variants of these sequences that have at least 80 %, at least 85 %, at least 90 %, at least 95%, at least 97.5%, at least 99% or at least 99.5% sequence identity with the amino acid sequence set forth in the respective template sequence of any one of SEQ ID Nos. 4-17. These variants include truncated versions of the sequences set forth in SEQ ID Nos. 4-17, for example N- or C-terminal truncations, with these truncations (i.e. the deleted amino acid stretches) typically being 1-10, preferably 1-5 amino acids in length. In such variants all the substitutions and invariable positions of the template sequence are retained.

In various embodiments, the amino acid sequence comprises as the first, N-terminal amino acid the residue M. If this is not present within the specific sequences disclosed herein, it may be artificially added, if desired, in particular to facilitate expression in a host cell. In various embodiments, the polypeptides disclosed herein may comprise an N-terminal extension comprising a 6xHis-tag, such as the amino acid sequence set forth in SEQ ID NO:21.

The invention further relates to the nucleic acid, in particular the isolated nucleic acid molecule, encoding the polypeptide as described above. The polypeptide may comprise in addition to the amino acid sequence defined herein other amino acid sequences that encode different (poly)peptides, for example (poly)peptide tags that are used or useful for detection or purification. Known tags include, without limitation, the 6xHis tag. These may be linked to the amino acid sequence defined herein by a linker peptide sequence, which is typically 1 to 20 amino acids in length, for example 2 to 10 amino acids, and may be a glycine-rich sequence. Such peptide tags are preferably attached to the N-terminal end of the amino acid sequence defined herein. These amino acid sequences are typically linked by peptide bonds and expressed as a single fusion protein.

In certain embodiments, the above defined nucleic acid molecules may be comprised in a vector, for example a cloning or expression vector. Generally, the nucleic acid molecules of the invention can also be part of a vector or any other kind of cloning vehicle, including, but not limited to a plasmid, a phagemid, a phage, a baculovirus, a cosmid, or an artificial chromosome. Generally, a nucleic acid molecule disclosed in this application may be "operably linked" to a regulatory sequence (or regulatory sequences) to allow expression of this nucleic acid molecule.

Such cloning vehicles can include, besides the regulatory sequences described above and a nucleic acid sequence of the present invention, replication and control sequences derived from a species compatible with the host cell that is used for expression as well as selection markers conferring a selectable phenotype on transformed or transfected cells. Large numbers of suitable cloning vectors are known in the art, and are commercially available.

In certain embodiments the nucleic acid molecules disclosed herein are comprised in a cloning vector. In some embodiments the nucleic acid molecules disclosed herein are comprised in an expression vector. The vectors may comprise regulatory elements for replication and selection markers. In certain embodiments, the selection marker may be selected from the group consisting of genes conferring ampicillin, kanamycin, chloramphenicol, tetracycline, blasticidin, spectinomycin, gentamicin, hygromycin, and zeocin resistance. In various other embodiments, the selection may be carried out using antibiotic-free systems, for example by using toxin/antitoxin systems, cer sequence, triclosan, auxotrophies or the like. Suitable methods are known to those skilled in the art.

The above-described nucleic acid molecule of the present invention, if integrated in a vector, must be integrated such that the polypeptide can be expressed. Therefore, a vector of the present invention comprises sequence elements which contain information regarding to transcriptional and/or translational regulation, and such sequences are "operably linked" to the nucleotide sequence encoding the polypeptide. An operable linkage in this context is a linkage in which the regulatory sequence elements and the sequence to be expressed are connected in a way that enables gene expression. The precise nature of the regulatory regions necessary for gene expression may vary among species, but in general these regions comprise a promoter which, in prokaryotes, contains both the promoter per se, i.e. DNA elements directing the initiation of transcription, as well as DNA elements which, when transcribed into RNA, will signal the initiation of translation. Such promoter regions normally include 5' non-coding sequences involved in initiation of transcription and translation, such as the -35/- 10 boxes and the Shine-Dalgarno element in prokaryotes or the TATA box, CAAT sequences, and 5'- capping elements in eukaryotes. These regions can also include enhancer or repressor elements as well as translated signal and leader sequences for targeting the native polypeptide to a specific compartment of a host cell.

In addition, the 3' non-coding sequences may contain regulatory elements involved in transcriptional termination, polyadenylation or the like. If, however, these termination sequences are not satisfactory functional in a particular host cell, then they may be substituted with signals functional in that cell.

In various embodiments, a vector comprising a nucleic acid molecule of the invention can therefore comprise a regulatory sequence, preferably a promoter sequence. In certain embodiments, the promoter is identical or homologous to promoter sequences of the host genome. In such cases endogenous polymerases may be capable to transcribe the nucleic acid molecule sequence comprised in the vector. In various embodiments, the promoter is selected from the group of weak, intermediate and strong promoters, preferably from weak to intermediate promoters.

In another preferred embodiment, a vector comprising a nucleic acid molecule of the present invention comprises a promoter sequence and a transcriptional termination sequence. Suitable promoters for prokaryotic expression are, for example, the araBAD promoter, the tet-promoter, the lacUV5 promoter, the CMV promo tor, the EF1 alpha promotor, the AOX1 promotor, the tac promotor, the T7promoter, or the lac promotor. Examples of promoters useful for expression in eukaryotic cells are the SV40 promoter or the CMV promoter. Furthermore, a nucleic acid molecule of the invention can comprise transcriptional regulatory elements, e.g., repressor elements, which allow regulated transcription and translation of coding sequences comprised in the nucleic acid molecule. Repressor element may be selected from the group consisting of the Lac-, AraC-, or MaIR-repressor.

The vector may be effective for prokaryotic or eukaryotic protein expression. In particular, the nucleic acid molecules of the present invention may be comprised in a vector for prokaryotic protein expression. Such vector sequences are constructed such that a sequence of interest can easily be inserted using techniques well known to those skilled in the art. In certain embodiments, the vector is selected from the group consisting of a pET-vector, a pBAD-vector, a pK184-vector, a pMONO-vector, a pSELECT-vector, pSELECT-Tag-vector, a pVITRO-vector, a pVIVO-vector, a pORF-vector, a pBLAST-vector, a pUO-vector, a pDUO-vector, a pZERO-vector, a pDeNy-vector, a pDRIVE-vector, a pDRIVE-SEAP-vector, a HaloTag^{®}Fusion- vector, a pTARGET^{™}-vector, a Flexi^{®}-vector, a pDEST-vector, a pHIL-vector, a pPIC-vector, a pMET-vector, a pPink-vector, a pLP -vector, a pTOPO-vector, a pBud-vector, a pCEP-vector, a pCMV-vector, a pDisplay-vector, a pEF-vector, a pFL-vector, a pFRT-vector, a pFastBac-vector, a pGAPZ-vector, a pIZ/V5-vector, a p3S-vector, a pIAR-vector, pSEC, pMS, a pSU2726-vector, a pLenti6 -vector, a pMIB-vector, a pOG-vector, a pOpti-vector, a pREP4-vector, a pRSET-vector, a p SCREEN- vector, a pSecTag-vector, a pTEFI -vector, a pTracer-vector, a pTrc-vector, a pUB6-vector, a pVAXI-vector, a pYC2-vector, a pYES2-vector, a pZeo-vector, a pcDNA-vector, a pFLAG-vector, a pTAC-vector, a pT7-vector, a gateway^{®}-vector, a pQE-vector, a pLEXY-vector, a pRNA-vector, a pPK-vector, a pUMVC-vector, a pLIVE-vector, a pCRUZ-vector, a Duet-vector, and other vectors or derivatives thereof.

The vectors of the present invention may be chosen from the group consisting of high, medium and low copy vectors.

The above described vectors of the present invention may be used for the transformation or transfection of a host cell in order to achieve expression of a polypeptide which is encoded by an above described nucleic acid molecule and comprised in the vector DNA. Thus, in a further aspect, the present invention also relates to a host cell comprising a vector or nucleic acid molecule as disclosed herein.

Also contemplated herein are host cells, which comprise a nucleic acid molecule as described herein integrated into their genomes. The skilled person is aware of suitable methods for achieving the nucleic acid molecule integration. For example, the molecule may be delivered into the host cells by means of liposome transfer or viral infection and afterwards the nucleic acid molecule may be integrated into the host genome by means of homologous recombination. In certain embodiments, the nucleic acid molecule is integrated at a site in the host genome, which mediates transcription of the peptide or protein of the invention encoded by the nucleic acid molecule. In various embodiments, the nucleic acid molecule further comprises elements which mediate transcription of the nucleic acid molecule once the molecule is integrated into the host genome and/or which serve as selection markers.

In certain embodiments, the nucleic acid molecule of the present invention is transcribed by a polymerase natively encoded in the host genome. In various embodiments, the nucleic acid molecule is transcribed by an RNA-polymerase which is non-native to the host genome. In such embodiments, the nucleic acid molecule of the present invention may further comprise a sequence encoding for a polymerase and/or the host genome may be engineered or the host cell may be infected to comprise a nucleic acid sequence encoding for an exogenous polymerase. The host cell may be specifically chosen as a host cell capable of expressing the gene. In addition or otherwise, in order to produce the polypeptide of the invention, the nucleic acid coding for it can be genetically engineered for expression in a suitable system. Transformation can be performed using standard techniques (Sambrook, J. et al. (2001), Molecular Cloning: A Laboratory Manual, 3rd Ed., Cold Spring Harbor Laboratory Press, Cold Spring Harbor, NY).

Prokaryotic or eukaryotic host organisms comprising such a vector for recombinant expression of the polypeptide as described herein form also part of the present invention. Suitable host cells can be prokaryotic cells. In certain embodiments the host cells are selected from the group consisting of gram positive and gram negative bacteria. In some embodiments, the host cell is a gram negative bacterium, such as E.coli. In certain embodiments, the host cell is E. coli, in particular E. coli BL21 (DE3) or other E. coli K12 or E. coli B834 derivatives. In further embodiments, the host cell is selected from the group consisting of Escherichia coli (E. coli), Pseudomonas, Serratia marcescens, Salmonella, Shigella (and other enterobacteriaceae), Neisseria, Hemophilus, Klebsiella, Proteus, Enter obacter, Helicobacter, Acinetobacter, Moraxella, Helicobacter, Stenotrophomonas, Bdellovibrio, Legionella, acetic acid bacteria, Bacillus, Bacilli, Carynebacterium, Clostridium, Listeria, Streptococcus, Staphylococcus, and Archaea cells. Suitable eukaryotic host cells are among others CHO cells, insect cells, fungi, yeast cells, e.g., Saccharomyces cerevisiae, S. pombe, Pichia pastoris.

In certain embodiments, the host cell is a prokaryotic cell, such as *E.coli,* in particular *E.coli* BL21 (DE3), E. coli BL21, E. coli K12, E. coli BLR, E. coli BL21 Al, E. coli BL21 pLysS, E. coli XL1 and E. coli DH5a. Further suitable *E.coli* strains include, but are not limited to DH1, DH5a, DM1, HB101, JMIOI-110, Rosetta(DE3)pLysS, SURE, TOP10, XLI-Blue, XL2-Blue and XLIO-Blue strains.

The transformed host cells are cultured under conditions suitable for expression of the nucleotide sequence encoding the polypeptide of the invention. In certain embodiments, the cells are cultured under conditions suitable for expression of the nucleotide sequence encoding a polypeptide of the invention and, optionally, its secretion.

For producing the recombinant polypeptide described herein, a vector of the invention can be introduced into a suitable prokaryotic or eukaryotic host organism by means of recombinant DNA technology (as already outlined above). For this purpose, the host cell is first transformed with a vector comprising a nucleic acid molecule according to the present invention using established standard methods (Sambrook, J. et al. (2001), supra). The host cell is then cultured under conditions, which allow expression of the heterologous DNA and thus the synthesis of the corresponding polypeptide. Subsequently, the polypeptide is recovered either from the cell or from the cultivation medium.

For expression of the polypeptides of the present invention several suitable protocols are known to the skilled person. The expression of a recombinant polypeptide of the present invention may be achieved by the following method comprising: (a) introducing a nucleic acid molecule or vector of the invention into a host cell, wherein the nucleic acid molecule or vector encodes the recombinant polypeptide; and (b) cultivating the host cell in a culture medium under conditions that allow expression of the recombinant polypeptide.

Step (a) may be carried out by using suitable transformation and transfection techniques known to those skilled in the art. These techniques are usually selected based on the type of host cell into which the nucleic acid is to be introduced. In some embodiments, the transformation may be achieved using electroporation or heat shock treatment of the host cell.

Step (b) may include a cultivation step that allows growth of the host cells. Alternatively, such step allowing growth of the host cells and a step that allows expression of the polypeptide may be performed separately in that the cells are first cultivated such that they grow to a desired density and then they are cultivated under conditions that allow expression of the polypeptide. The expression step can however still allow growth of the cells.

The method may further include a step of recovering the expressed polypeptide. The polypeptide may be recovered from the growth medium, if it is secreted, or from the cells or both. The recovery of the polypeptide may include various purification steps.

Generally, any known culture medium suitable for growth of the selected host may be employed in this method. In various embodiments, the medium is a rich medium or a minimal medium. Also contemplated herein is a method, wherein the steps of growing the cells and expressing the peptide or protein comprise the use of different media. For example, the growth step may be performed using a rich medium which is replaced by a minimal medium in the expression step. In certain cases, the medium is selected from the group consisting of LB medium, TB medium, 2YT medium, synthetical medium and minimal medium.

In various embodiments, the method also encompasses the purification the recombinant polypeptide, wherein the recombinant polypeptide is purified using a method selected from affinity chromatography, ion exchange chromatography, reverse phase chromatography, size exclusion chromatography, and combinations thereof.

In a further aspect, the present invention relates to the use of a vector or nucleic acid molecule as disclosed herein for the expression of a recombinant polypeptide. In some embodiments, the vector is used for the expression and optionally secretion of a recombinant polypeptide. The expression or expression and secretion may be achieved using the method described herein.

A method for expression of a recombinant polypeptide using the above-described nucleic acid molecules may comprise the steps of:
(a) introducing a nucleic acid molecule or a vector as described above into a suitable host cell, wherein the nucleic acid molecule or vector encodes the recombinant polypeptide; and
(b) cultivating the host cell in a culture medium under conditions that allow expression of the recombinant polypeptide.

The invention further relates to methods for the proteolytic cleavage of a substrate peptide or polypeptide. These substrate polypeptides typically comprise the specific TEV protease recognition and cleavage site with the amino acid sequence set forth in SEQ ID NO:18 (ENLYFQ X), wherein X can be any amino acid, preferably G, S, V, I, L, K, R, T, or E, for example G or V. While the wildtype TEV protease is highly specific for G as residue X, at the so-called P1' position, it is preferred that the variants allow other amino acids at this position without affecting catalytic activity and efficiency. As the C-terminal amino acid at position X of the motif remains in the cleaved C-terminal part, and the TEV cleavage site if often located on the N-terminus of a polypeptide of interest, it is advantageous if the TEV variant can accommodate different amino acids in this position to avoid an undesired substitution in this position. The variants of TEV protease disclosed herein have been found to have an altered substrate specificity with respect to this position and thus represent an improvement over TEV wildtype.

In the methods for the cleavage of a substrate polypeptide, the TEV protease variant of the invention is contacted with the substrate polypeptide under conditions that allow the cleavage of the polypeptide. Such conditions are generally known to those skilled in the art and may include specific buffer conditions and temperature control. After the cleavage reaction has occurred, the cleaved polypeptide may be subjected to further purification steps in order to separate it from the other fragment and/or the protease. Suitable purification protocols are known to those skilled in the art and include chromatographic methods, such as gel filtration, ion exchange or affinity chromatography, without being limited thereto.

In a further aspect, the present invention is also directed to the use of the polypeptides disclosed herein for cleavage of a substrate polypeptide as defined herein above.

In all methods and uses disclosed herein, the substrate polypeptide may be a fusion protein, preferably a non-natural fusion protein. Such fusion proteins typically comprise at least two different protein parts, domains or regions that may be connected by a linker that includes the TEV protease site. After expression and isolation, the different parts, domains or regions may thus be separated from each other by subjecting the fusion protein to a cleavage reaction with TEV protease variants and subsequent purification/separation.

The present invention further encompasses kits and compositions that comprise the polypeptides, nucleic acids, vectors and/or host cells of the invention. Such kits may further comprise instructions for use and/or buffers and other materials to allow directed use and application of the polypeptides.

### EXAMPLES

### Materials and methods

### Pre-culture

Individual clones were picked and inoculated in sterile 96-well plates, referred to as master plates, containing 100 µL of LB media, supplemented with 34 ug/mL Chloramphenicol + 30 ug/mL Kanamycin, per well. Plates were sealed with parafilm and incubated at 37 °C, 250 rpm and 80 % relative humidity in a humidity shaker overnight.

### Main culture

Next day plates were replicated with cryo replicator into new 96-deep well plates containing 300 µL of TB media supplemented with 34 µg/mL Chloramphenicol, 30 µg/mL Kanamycin and 2 mM MgCl₂. Plates were incubated at 37 °C, 250 rpm and 80 % relative humidity approximately 5 hours. When OD600 reached 0.6- 0.8., in each well 100 µL of TB media supplemented with 34 µg/mL Cm + 30 µg/mL Km + 2 mM MgCl₂ and IPTG in a final concentration of 0.1 mM were added. Plates were further incubated for 19 h, 250 rpm, 80 % relative humidity at 25 °C.

### Cell disruption

Plates were centrifuged (Eppendorf 5810R centrifuge, Germany) for 30 min, 3500 rpm at 4 °C and supernatant was discarded. In each cell pellet 150 µL of Bug Buster solution was added using multidrop dispenser (Multidrop Combi Reagent Dispenser, Thermo Scientific, USA) and plates were shaken in a plate shaker until the pellet was resuspended (few minutes). After that, plates were incubated in a humidity shaker for 20 min, 25 °C, 250 rpm. In the next step plates were centrifuged for 40 min, 3500 rpm at 4 °C, obtaining lysate solution.

### Activity assay

20 µL of the lysate was transferred from the 96-deep well plate using liquid handler robotic station (Freedom EVO, Tecan, Switzerland) to a microplate already containing 80 µL of high purity water (1/5 dilution). Afterwards, from the 1/5 dilution microplate, 50 µL were transferred by liquid handler robotic station to a new microplate containing 50 µL of assay buffer 2X (100 mM Tris-HCl, 1 mM EDTA) (1/10 dilution). Next, 25 µL of 1/10 dilution were transferred to empty opaque microplates, 25 µL of previously prepared substrate solution (2 µL Substrate (diluted in DMSO, 1 mM), 2 µL DTT (1M) in assay buffer) was added and fluorescence was measured immediately in plate reader (SPECTRAMax Plus 384, Molecular Devices, USA). Excitation was performed at a wavelength of 340 nm and emission was recorded at 490 nm.

### Cloning of the variants

P1534 plasmid (pSF593 plasmid vector with N-terminal 6xHis-tag and TEV double mutant (SEQ ID NO:3) insert with N-terminal tag (SEQ ID NO:21)) was digested with restriction enzymes BamHI-HF and Hindlll-HF (NEB, USA). Cloning was performed with Gibson Assembly^{®} (NEB, USA) and reactions were transformed into Escherichia coli XL2-Blue Ultracompetent Cells (Agilent, USA). The circular plasmids were retrieved through miniprep and sequenced. Once the sequences were confirmed to be correct, they were transformed together with pRIL plasmid in E. coli BL21 cells following the protocol.

### Expression of variants in flasks

Growing and expression of variants in flask was performed in LB-media using a pre- and main culture cultivation. The pre-cultures were inoculated with one colony from plates in 5 mL LB media, supplemented with 30 µg/mL Kanamycin and 34 µg/mL chloramphenicol. Cells were grown over night at 30°C, 180 rpm.

Main cultures in TB-media were set in 250 mL Erlenmeyer baffled flasks in a total volume of 25 mL media, supplemented with 30 µg/mL Kanamycin, 34 µg/mL chloramphenicol and 2 mM MgCl2. Main cultures were inoculated with pre-culture corresponding to an optical density (600 nm) of ~ 0.1 from pre-culture and cells were incubated at 30°C, 180 rpm, until and cell density of 0.6-0.8 incubator was set to 25°C and induced with 0.1 mM IPTG incubation was continued for 19 h, 120 rpm, at 25°C.

Activity was measured with TEV Protease Activity Assay Kit from Abcam (UK).

### Thermostability evaluation

To evaluate stability in the obtained mutants, dilution 1/5 of Bugbuster lysate (5 µL of the lysate in 20 µL of MilliQ water) was heated 10 min at 45 °C. Afterwards it was introduced in ice for 10 min and chilled at room temperature 5 min. 5 µL of dilution 1/5 were added to 95 µL of assay buffer (dilution 1/100) and 25 µL of that dilution were mixed with 25 µL of substrate solution to evaluate activity.

### Fluorogenic peptides

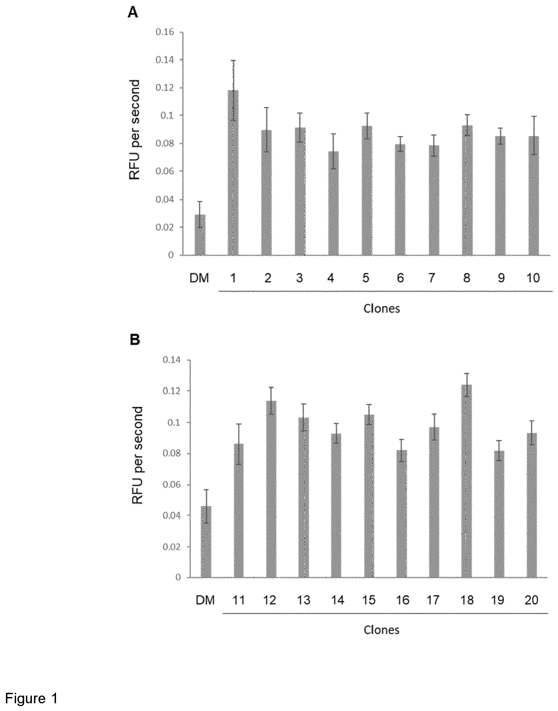

Peptides were designed attached to fluorophore EDANS (5-[(2-Aminoethyl)amino]naphthalene-1-sulfonic acid) and quencher Dabcyl (4-([4'-dimethylamino)phenyl]azo)benzoyl) to give a fluorometric response when cleaved. Each peptide was synthetized by Pepscan (The Netherlands) with {E(Edans)} = Edans attached to the N-terminal glutamic acid sidechain, {K(Dabcyl)} = Dabcyl attached to the C-terminal lysine sidechain of the substrate ENLYFQXGGK (X= G, V, I, L, K, R, T or E; SEQ ID NO:22). Excitation was performed at a wavelength of 340 nm and emission was recorded at 490 nm. Figure 1.

### Activity assay from flask expression

2 mL aliquot of cultivation broth were centrifuged (10 min, 15000 rpm) and the supernatant is discarded. The cell pellet is resuspended in 160 µL of Bug Buster solution and incubating (20 min, 25 °C, 600 rpm) afterwards. Afterwards, centrifugation (20 min, 16000 rcf) is performed and 100 µL of the supernatant of the lysate is placed on ice. 5 µL of the lysate was diluted in 20 µL MQ H₂O (DF 1/5). 5 µL of this dilution is mixed in 45 µL of assay buffer 2x (100 mM Tris-HCl, 1 mM EDTA) (DF 1/50). 25 µL of the lysate (DF 1/50) of each sample are pipetted into the 96 well plate as triplicate. Afterwards, 25 µL of the prepared substrate solution (2 µL Substrate (diluted in DMSO, 1 mM), 2 µL DTT (1M) in assay buffer) are added swiftly to each well and measurement started immediately in plate reader (SPECTRAMax Plus 384, Molecular Devices, USA). Excitation was performed at a wavelength of 340 nm and emission was recorded at 490 nm.

### FuncLib design

FuncLib is an automated method for designing multipoint mutations at enzyme active sites using phylogenetic analysis and Rosetta design calculations. In order to obtain the FuncLib variants, the positions selected were studied by the algorithm and from the pool of 50 variants, 26 were chosen for experiments after close inspection of each mutation.

### Microplate expression of FuncLib variants and activity assay

5 individual colonies of each variant were evaluated following the protocol developed at EvoEnzyme for microplate growing. P1rev was used as positive control and for comparison purposes. Activity was evaluated with the 8 substrates, upscaling consequently the total volume used in dilution steps to have enough for all measurements.

**Table 1: Overview of the variants generated and tested (substitutions relative to SEQ ID NO:1)**

| | SEQ ID NO: | | | | | | | | |
|---|---|---|---|---|---|---|---|---|---|
| DM | 3 | S153N | R203G | | | | | | |
| 1 | 4 | T22V | T30A | D148N | R203G | M218F | | | |
| 2 | 6 | T22I | T30A | R203G | M218F | | | | |
| 3 | 4 | T22V | T30A | D148N | R203G | M218F | | | |
| 4 | 5 | T22S | T30A | D148N | R203G | M218F | | | |
| 5 | 11 | T22I | T30S | R203G | M218F | | | | |
| 6 | 5 | T22S | T30A | D148N | R203G | M218F | | | |
| 7 | 6 | T22I | T30A | R203G | M218F | | | | |
| 8 | 7 | T22I | T30A | D148R | R203G | M218F | | | |
| 9 | 5 | T22S | T30A | D148N | R203G | M218F | | | |
| 10 | 5 | T22S | T30A | D148N | R203G | M218F | | | |
| 11 | 8 | T22Y | T30S | D148N | R203G | M218F | | | |
| 12 | 5 | T22S | T30A | D148N | R203G | M218F | | | |
| 13 | 12 | T22I | T30S | D148R | R203G | M218F | | | |
| 14 | 12 | T22I | T30S | D148R | R203G | M218F | | | |
| 15 | 8 | T22Y | T30S | D148N | R203G | M218F | | | |
| 16 | 11 | T22I | T30S | R203G | M218F | | | | |
| 17 | 12 | T22I | T30S | D148R | R203G | M218F | | | |
| 18 | 5 | T22S | T30A | D148N | R203G | M218F | | | |
| 19 | 9 | T22I | T30S | D148Y | R203G | M218F | | | |
| 20 | 10 | T22Y | T30S | D148R | R203G | M218F | | | |
| P1 | 13 | Q74L | S135G | I138T | S153N | R203G | | | |
| P3 | 14 | Q74L | S135G | I138H | S153N | R203G | L60W | F5C | L210C |
| P4* | 15 | Q74L | S135G | I138H | S153N | R203G | L60W | F5C | L210C |
| P5* | 16 | Q74L | S135G | I138H | S153N | R203G | L60W | F5C | L210C |
| P1rev | 17 | Q74L | S135G | I138T | R203G | | | | |

| | | | | | | | | | |
|---|---|---|---|---|---|---|---|---|---|
| 1 = 3; 2 = 7; 4 = 6 = 9 = 10 = 12 = 18; 5 = 16; 11 = 15; 13 = 14 = 17 *P4 additionally comprised S200K, L76P, C130T and A206T *P5 additionally comprised S200K, L76P, C130T, A206T, T118E, T113G, D78E, and D127E | | | | | | | | | |

### Example 1: Screening assay

Standard TEV (SEQ ID NO:1), a known double mutant (DM; SEQ ID NO:3) and P8ref (negative control; SEQ ID NO:23) were grown in microplate format and TEV activity was assessed with Gly peptide (Edans-ENLYFQGGGK-Dabcyl); SEQ ID NO:19) using original activity assay protocols for flask expression and also using the modified activity assay protocol for 96-well expression described above. Fluorescence activity levels were significantly higher when modified protocol was employed, Figure 1. Also, activity was sufficiently high to serve as a control screening.

Applying the same modified protocol, screening assay was validated with standard TEV (SEQ ID NO:1) and Gly peptide (SEQ ID NO:19). 58 clones of standard TEV were assayed and an acceptable coefficient of variation (CV) of 15 % was obtained (data not shown).

Prior to library screening standard TEV, DM and p8ref (negative control) were tested with the Val peptide (SEQ ID NO:20), the target for further screening, in order to estimate screening expectations. We observed consistent activity, albeit low, with the DM (SEQ ID NO:3), while with our parental type standard TEV (SEQ ID NO:1) no activity was detected.

A library of 320 individual mutants was transformed to the BL21 *E. coli* competent cells using pRIL as co-expression plasmid. Applying modified growing/assay protocol 896 individual clones, inoculated in 11 microplates, were screened with both, Gly and Val peptides, respectively. In each plate column 6 was inoculated with standard TEV (SEQ ID NO:1), while column 7 was inoculated with DM (SEQ ID NO:3), to allow reliable comparisons. In addition, in each plate a negative control was used.

Considering parental type did not show activity against Val peptide, clones in the library were compared to the DM (SEQ ID NO:3). 119 clones showed higher activity than the CV threshold (21 %), while 78 clones displayed more than 2-fold improvement over DM (data not shown). After careful data analysis, 20 clones were selected for the rescreening process.

Selected clones were regrown, plasmids were extracted and BL21 *E. coli* was re-transformed. 20 clones, together with standard TEV and DM were inoculated in 2 microplates, having each clone picked 8 times in a single column (Plate 1 - clones 1 to 10; Plate 2 - clones 11 to 20). All clones reported herein were expressed with an N-terminal 6xHis-tag and a G-rich linker sequence (SEQ ID NO:21). Again, in each plate column 6 was inoculated with standard TEV, while column 7 was inoculated with DM. Applying the same modified growing/assay protocol, mutants were screened with both peptides.

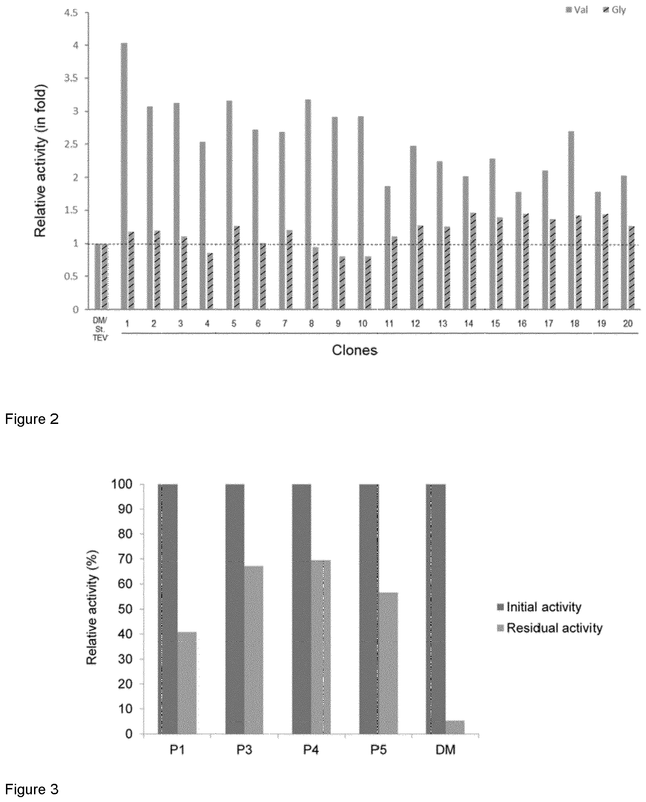

All clones showed significantly higher activity with the Val peptide than the DM confirming our screening results, Figure 1. Furthermore, plates were also screened with Gly peptide and all 20 clones retained their respective activities, Figure 2.

Afterwards, all 20 clones were sent to sequencing and the results are depicted in Table 1. All clones additionally comprised the N-terminal sequence set forth in SEQ ID NO:21 to allow purification. As expected, there were repetition in the mutants due to the library nature. In total, 9 different clones were identified. Original parental type already contained mutation R203G. Mutation M218F was present in all clones and was found to be important for altered activity towards Val peptide. In position 148, we observed 3 changes (N, R and Y). However, it is the only position that was not changed in some of the clones and still good activity was observed. In position 30, there were two changes, A and S, where clones with A showed higher activity in general. Lastly, in position 22, we identified all 4 possible changes (V, I, Sand Y).

### Example 2: Rational design of TEV variants

Positions selected to be fixed (maintain unaltered) in order to avoid activity loss were the following: 17, 46, 68, 77, 81, 151, 153, 203 and 219. The quality of the alignment was optimal, as there were a lot of sequences with a high coverage and also the sequence identity was low enough to have good diversity but not to be too far from TEV protease sequence. Different mutants were obtained with the regular PROSS algorithm and mutations close to important residues were deselected. Non redundant mutants were selected.

Mutants were cloned and expressed in flask for activity and thermostability studies. Four mutants P1, P3, P4 and P5 were found to surpassed DM's (SEQ ID NO:3) activity, the rest had similar values as standard TEVp (SEQ ID NO:1) and the mutant P8ref (SEQ ID NO: 23) was found to have no activity at all. It was verified that the specific activity of the variants has been increased by ruling out that the observed effects are due to differences in expression.

In order to select the best option for FuncLib, the thermostability was evaluated (Figure 3). All of the mutants tested were more thermostable than DM.

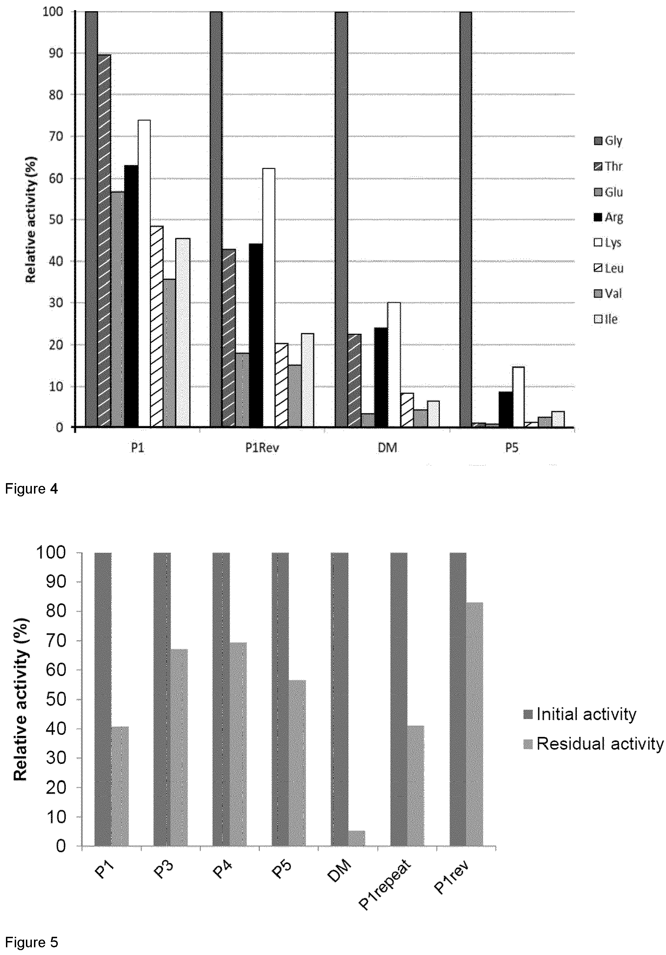

All of the variants were tested for activity. P1 outperformed DM but the P5 mutant resulted to be the worst candidate compared to P1. The P1 mutant was cloned again without the S153N mutation (P1rev) and we assayed the variants with the different substrates where the G in SEQ ID NO:19 following the TEV recognition sequence was varied and replaced by other amino acids as indicated (Figure 4). P1rev was not as good as P1 but it was much better than DM. P5 was not the best candidate according to its activity in comparison with other variants, so we tested P1rev thermostability to evaluate if it was stable enough for further rational optimization.

Thermostability results showed that P1rev outperformed the rest of the variants, including patent protected DM, presenting P1rev as the best candidate further optimization (Figure 5). This mutant was more stable, with a less selective activity profile. According to further analysis of the mutant, it seems that mutations have allowed stabilization of the loop through new polar contacts within the amino acids in the surroundings, allowing the thermostability change and possibly causing an overall stabilization of the protein leading to an improvement in activity.

The experiments conducted allowed to obtain a variant with higher stability than DM and standard TEVp Said mutant was found to have better activity with the fluorogenic modified substrates and better thermal stability, making it an ideal candidate for future protein engineering studies.

All documents cited herein, are hereby incorporated by reference in their entirety. The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. In addition, where features or aspects of the invention are described in terms of Markush groups, those skilled in the art will recognize that the invention is also thereby described in terms of any individual member or subgroup of members of the Markush group. Further embodiments of the invention will become apparent from the following claims.

## Claims

1. Polypeptide comprising an amino acid sequence that has at least 80 %, at least 85 %, at least 90 %, at least 95%, at least 97.5%, at least 99% or at least 99.5% sequence identity over its entire length with the amino acid sequence set forth in SEQ ID NO:1 and comprises one or more amino acid substitution(s) selected from the group consisting of: M218F, T22V/I, T30A, D148N/R, Q74L, and S135G, wherein the positions 17, 68, 77, 219 (scaffold), 46, 81 and 151 (catalytic triad) are invariable, wherein the positional numbering is according to SEQ ID NO:1, or a functional fragment thereof.

2. The polypeptide of claim 1, wherein the polypeptide further comprises any one or more of the amino acid substitution(s) I138T, S153N and R203G, wherein the positional numbering is according to SEQ ID NO:1.

3. The polypeptide of claim 1 or 2, wherein the amino acid sequence that has at least 80 %, at least 85 %, at least 90 %, at least 95%, at least 97.5%, at least 99% or at least 99.5% sequence identity over its entire length with the amino acid sequence set forth in SEQ ID NO:1 at its C-Terminus retains the deletion of amino acids 237-242 relative to the TEV protease wildtype sequence set forth in SEQ ID NO:2.

4. The polypeptide of any one of claims 1 to 3, wherein the functional fragment is at least 202 amino acids in length and comprises amino acid residues corresponding to those at positions 17 to 218 of SEQ ID NO:1.

5. The polypeptide of any one of claims 1 to 4, wherein the polypeptide comprises
(1) the amino acid substitution M218F and optionally any one or more of T22V/I, T30A, D148N/R, Q74L, and S135G;
(2) the amino acid substitution M218F and optionally any one or more of T22V/I, T30A, D148N/R, Q74L, S135G, I138T, S153N and R203G;
(3) the amino acid substitution M218F and any two, any three, any four, or all five of T22V/I, T30A, D148N/R, Q74L, and S135G;
(4) the amino acid substitution M218F and any two, any three, any four, any five, any six, any seven, or all 8 of T22V/I, T30A, D148N/R, Q74L, S135G, I138T, S153N and R203G;
(5) the amino acid substitutions M218F and T22V/I and optionally any one, any two, any three, or all four of T30A, D148N/R, Q74L, and S135G;
(6) the amino acid substitutions M218F and T22V/I and optionally any one, any two, any three, any four, any five, any six, or all seven of T30A, D148N/R, Q74L, S135G, I138T, S153N and R203G;
(7) the amino acid substitutions M218F and T30A and optionally any one, any two, any three, or all four of T22V/I, D148N/R, Q74L, and S135G;
(8) the amino acid substitutions M218F and T30A and optionally any one, any two, any three, any four, any five, any six, or all seven of T22V/I, D148N/R, Q74L, S135G, I138T, S153N and R203G;
(9) the amino acid substitutions M218F and D148N/R and optionally any one, any two, any three, or all four of T22V/I, T30A, Q74L, and S135G;
(10) the amino acid substitutions M218F and D148N/R and optionally any one, any two, any three, any four, any five, any six, or all seven of T22V/I, T30A, Q74L, S135G, I138T, S153N and R203G;
(11) the amino acid substitutions M218F and Q74L and optionally any one, any two, any three, or all four of T22V/I, T30A, D148N/R, and S135G;
(12) the amino acid substitutions M218F and Q74L and optionally any one, any two, any three, any four, any five, any six, or all seven of T22V/I, T30A, D148N/R, S135G, I138T, S153N and R203G;
(13) the amino acid substitutions M218F and S135Gand optionally any one, any two, any three, or all four of T22V/I, T30A, D148N/R, and Q74L;
(14) the amino acid substitutions M218F and S135G and optionally any one, any two, any three, any four, any five, any six, or all seven of T22V/I, T30A, D148N/R, Q74L, I138T, S153N and R203G;
(15) the amino acid substitutions M218F and I138T and optionally any one, any two, any three, any four, any five, any six, or all seven of T22V/I, T30A, D148N/R, Q74L, S135G, S153N and R203G;
(16) the amino acid substitutions M218F and S153N and optionally any one, any two, any three, any four, any five, any six, or all seven of T22V/I, T30A, D148N/R, Q74L, S135G, I138T and R203G;
(17) the amino acid substitutions M218F and R203G and optionally any one, any two, any three, any four, any five, any six, or all seven of T22V/I, T30A, D148N/R, Q74L, S135G, I138T and S153N;
(18) the amino acid substitutions M218F, S153N and R203G and optionally any one, any two, any three, any four, any five, or all six of T22V/I, T30A, D148N/R, Q74L, S135G and I138T;
(19) the amino acid substitution T22V/I and optionally any one or more of T30A, D148N/R, Q74L, S135G, and M218F;
(20) the amino acid substitution T22V/I and optionally any one or more of T30A, D148N/R, Q74L, S135G, I138T, S153N, R203G and M218F;
(21) the amino acid substitution T22V/I and any two, any three, any four, or all five of T30A, D148N/R, Q74L, S135G, and M218F;
(22) the amino acid substitution T22V/I and any two, any three, any four, any five, any six, any seven, or all eight of T30A, D148N/R, Q74L, S135G, I138T, S153N, R203G, and M218F;
(23) the amino acid substitutions T22V/I and T30A and optionally any one, any two, any three, r all four of M218F, D148N/R, Q74L, and S135G;
(24) the amino acid substitution T22V/I and T30A and optionally any one, any two, any three, any four, any five, any six, or all seven of M218F, D148N/R, Q74L, S135G, I138T, S153N and R203G;
(25) the amino acid substitutions T22V/I and D148N/R and optionally any one, any two, any three, or all four of M218F, T30A, Q74L, and S135G;
(26) the amino acid substitution T22V/I and D148N/R and optionally any one, any two, any three, any four, any five, any six, or all seven of M218F, T30A, Q74L, S135G, I138T, S153N and R203G;
(27) the amino acid substitutions T22V/I and Q74L and optionally any one, any two, any three, or all four of M218F, T30A, D148N/R, and S135G;
(28) the amino acid substitutions T22V/I and Q74L and optionally any one, any two, any three, any four, any five, any six, or all seven of M218F, T30A, D148N/R, S135G, I138T, S153N and R203G;
(29) the amino acid substitutions T22V/I and S135G and optionally any one, any two, any three, or all four of M218F, T30A, D148N/R, and Q74L;
(30) the amino acid substitutions T22V/I and S135G and optionally any one, any two, any three, any four, any five, any six, or all seven of M218F, T30A, D148N/R, Q74L, I138T, S153N and R203G;
(31) the amino acid substitutions T22V/I and I138T and optionally any one, any two, any three, any four, any five, any six, or all seven of M218F, T30A, D148N/R, Q74L, S135G, S153N and R203G;
(32) the amino acid substitutions T22V/I and S153N and optionally any one, any two, any three, any four, any five, any six, or all seven of M218F, T30A, D148N/R, Q74L, S135G, I138T and R203G;
(33) the amino acid substitutions T22V/I and R203G and optionally any one, any two, any three, any four, any five, any six, or all seven of M218F, T30A, D148N/R, Q74L, S135G, I138T and S153N;
(34) the amino acid substitutions T22V/I, S153N and R203G and optionally any one, any two, any three, any four, any five, or all six of M218F, T30A, D148N/R, Q74L, S135G and I138T;
(35) the amino acid substitution T30A and optionally any one or more of T22V/I, D148N/R, Q74L, S135G, and M218F;
(36) the amino acid substitution T30A and optionally any one or more of T22V/I, D148N/R, Q74L, S135G, I138T, S153N, R203G and M218F;
(37) the amino acid substitution T30A and any two, any three, any four, or all five of T22V/I, D148N/R, Q74L, S135G, and M218F;
(38) the amino acid substitution T30A and any two, any three, any four, any five, any six, any seven, or all eight of T22V/I, D148N/R, Q74L, S135G, I138T, S153N, R203G, and M218F;
(39) the amino acid substitutions T30A and D148N/R and optionally any one, any two, any three, or all four of M218F, T22V/I, Q74L, and S135G;
(40) the amino acid substitution T30A and D148N/R and optionally any one, any two, any three, any four, any five, any six, or all seven of M218F, T22V/I, Q74L, S135G, I138T, S153N and R203G;
(41) the amino acid substitutions T30A and Q74L and optionally any one, any two, any three, or all four of M218F, T22V/I, D148N/R, and S135G;(42) the amino acid substitutions T30A and Q74L and optionally any one, any two, any three, any four, any five, any six, or all seven of M218F, T22V/I, D148N/R, S135G, I138T, S153N and R203G;
(43) the amino acid substitutions T30A and S135G and optionally any one, any two, any three, or all four of M218F, T22V/I, D148N/R, and Q74L;
(44) the amino acid substitutions T30A and S135G and optionally any one, any two, any three, any four, any five, any six, or all seven of M218F, T22V/I, D148N/R, Q74L, I138T, S153N and R203G;
(45) the amino acid substitutions T30A and I138T and optionally any one, any two, any three, any four, any five, any six, or all seven of M218F, T22V/I, D148N/R, Q74L, S135G, S153N and R203G;
(46) the amino acid substitutions T30A and S153N and optionally any one, any two, any three, any four, any five, any six, or all seven of M218F, T22V/I, D148N/R, Q74L, S135G, I138T and R203G;
(47) the amino acid substitutions T30A and R203G and optionally any one, any two, any three, any four, any five, any six, or all seven of M218F, T22V/I, D148N/R, Q74L, S135G, I138T and S153N;
(48) the amino acid substitutions T30A, S153N and R203G and optionally any one, any two, any three, any four, any five, or all six of M218F, T22V/I, D148N/R, Q74L, S135G and I138T;
(49) the amino acid substitution D148N/R and optionally any one or more of T22V/I, T30A, Q74L, S135G, and M218F;
(50) the amino acid substitution D148N/R and optionally any one or more of T22V/I, T30A, Q74L, S135G, I138T, S153N, R203G and M218F;
(51) the amino acid substitution D148N/R and any two, any three, any four, or all five of T22V/I, T30A, Q74L, S135G, and M218F;
(52) the amino acid substitution D148N/R and any two, any three, any four, any five, any six, any seven, or all eight of T22V/I, T30A, Q74L, S135G, I138T, S153N, R203G, and M218F;
(53) the amino acid substitutions D148N/R and Q74L and optionally any one, any two, any three, or all four of M218F, T22V/I, T30A, and S135G;
(54) the amino acid substitutions D148N/R and Q74L and optionally any one, any two, any three, any four, any five, any six, or all seven of M218F, T22V/I, T30A, S135G, I138T, S153N and R203G;
(55) the amino acid substitutions D148N/R and S135G and optionally any one, any two, any three, or all four of M218F, T22V/I, T30A, and Q74L;
(56) the amino acid substitutions D148N/R and S135G and optionally any one, any two, any three, any four, any five, any six, or all seven of M218F, T22V/I, T30A, Q74L, I138T, S153N and R203G;
(57) the amino acid substitutions D148N/R and I138T and optionally any one, any two, any three, any four, any five, any six, or all seven of M218F, T22V/I, T30A, Q74L, S135G, S153N and R203G;
(58) the amino acid substitutions D148N/R and S153N and optionally any one, any two, any three, any four, any five, any six, or all seven of M218F, T22V/I, T30A, Q74L, S135G, I138T and R203G;
(59) the amino acid substitutions D148N/R and R203G and optionally any one, any two, any three, any four, any five, any six, or all seven of M218F, T22V/I, T30A, Q74L, S135G, I138T and S153N;
(60) the amino acid substitutions D148N/R, S153N and R203G and optionally any one, any two, any three, any four, any five, or all six of M218F, T22V/I, T30A, Q74L, S135G and I138T;
(61) the amino acid substitution Q74L and optionally any one or more of T22V/I, T30A, D148N/R, S135G, and M218F;
(62) the amino acid substitution Q74L and optionally any one or more of T22V/I, T30A, D148N/R, S135G, I138T, S153N, R203G and M218F;
(63) the amino acid substitution Q74L and any two, any three, any four, or all five of T22V/I, T30A, D148N/R, S135G, and M218F;
(64) the amino acid substitution Q74L and any two, any three, any four, any five, any six, any seven, or all eight of T22V/I, T30A, D148N/R, S135G, I138T, S153N, R203G, and M218F;
(65) the amino acid substitutions Q74L and S135G and optionally any one, any two, any three, or all four of M218F, T22V/I, T30A, and D148N/R;
(66) the amino acid substitutions Q74L and S135G and optionally any one, any two, any three, any four, any five, any six, or all seven of M218F, T22V/I, T30A, D148N/R, I138T, S153N and R203G;
(67) the amino acid substitutions Q74L and I138T and optionally any one, any two, any three, any four, any five, any six, or all seven of M218F, T22V/I, T30A, D148N/R, S135G, S153N and R203G;
(68) the amino acid substitutions Q74L and S153N and optionally any one, any two, any three, any four, any five, any six, or all seven of M218F, T22V/I, T30A, D148N/R, S135G, I138T and R203G;
(69) the amino acid substitutions Q74L and R203G and optionally any one, any two, any three, any four, any five, any six, or all seven of M218F, T22V/I, T30A, D148N/R, S135G, I138T and S153N;
(70) the amino acid substitutions Q74L, S153N and R203G and optionally any one, any two, any three, any four, any five, or all six of M218F, T22V/I, T30A, D148N/R, S135G and I138T;
(71) the amino acid substitution S135G and optionally any one or more of T22V/I, T30A, D148N/R, Q74L, and M218F;
(72) the amino acid substitution S135G and optionally any one or more of T22V/I, T30A, D148N/R, Q74L, I138T, S153N, R203G and M218F;
(73) the amino acid substitution S135G and any two, any three, any four, or all five of T22V/I, T30A, D148N/R, Q74L, and M218F;
(74) the amino acid substitution S135G and any two, any three, any four, any five, any six, any seven, or all eight of T22V/I, T30A, D148N/R, Q74L, I138T, S153N, R203G, and M218F;
(75) the amino acid substitutions S135G and I138T and optionally any one, any two, any three, any four, any five, any six, or all seven of M218F, T22V/I, T30A, D148N/R, Q74L, S153N and R203G;
(76) the amino acid substitutions S135G and S153N and optionally any one, any two, any three, any four, any five, any six, or all seven of M218F, T22V/I, T30A, D148N/R, Q74L, I138T and R203G;
(77) the amino acid substitutions S135G and R203G and optionally any one, any two, any three, any four, any five, any six, or all seven of M218F, T22V/I, T30A, D148N/R, Q74L, I138T and S153N;
(78) the amino acid substitutions S135G, S153N and R203G and optionally any one, any two, any three, any four, any five, or all six of M218F, T22V/I, T30A, D148N/R, Q74L and I138T;
(79) the amino acid substitutions M218F, T22V/I and T30A and any one, any two, or all three of D148N/R, Q74L, and S135G;
(80) the amino acid substitutions M218F, T22V/I and D148N/R and any one, any two, or all three of T30A, Q74L, and S135G;
(81) the amino acid substitutions M218F, T30A and D148N/R and any one, any two, or all three of T22V/I, Q74L, and S135G;
(82) the amino acid substitutions T22V/I, T30A and D148N/R and any one, any two, or all three of M218F, Q74L, and S135G;
(83) the amino acid substitutions M218F, T22V/I, T30A and D148N/R and any one, or both of Q74L and S135G; or
(84) the amino acid substitutions M218F, T22V/I, T30A and D148N/R and any one, any two, any three, any four, or all five of Q74L, S135G, I138T, S153N, and R203G.

6. The polypeptide of any one of claims 1 to 5, wherein
(a) T22V/I is T22V or T22V/I is T22I; and/or
(b) D148N/R is D148R or D148N/R is D148N.

7. The polypeptide of any one of claims 1 to 6, wherein the polypeptide is an isolated polypeptide.

8. The polypeptide of any one of claims 1 to 7, wherein
(a) the polypeptide is up to 236 amino acids in length;
(b) the polypeptide has protease activity;
(c) the polypeptide comprises or consists of the amino acid sequence set forth in any one of SEQ ID Nos. 4 to 17.

9. Composition comprising the polypeptide of any one of claims 1 to 8.

10. Nucleic acid encoding the polypeptide of any one of claims 1 to 8.

11. Vector comprising a nucleic acid molecule according to claim 10.

12. Host cell comprising a nucleic acid molecule according to claim 10 or a vector according to claim 11, wherein preferably the host cell is a prokaryotic host cell.

13. Method for the cleavage of a substrate polypeptide comprising the amino acid sequence motif set forth in SEQ ID NO:18 (ENLYFQX), comprising contacting the substrate polypeptide with the polypeptide of any one of claims 1 to 8 under conditions that allow the cleavage of the polypeptide; and optionally purifying the cleaved polypeptide.

14. Use of the polypeptide of any one of claims 1 to 8 for cleavage of a substrate polypeptide comprising the amino acid motif of SEQ ID NO:18.

15. The method or use of claims 13 and 14, wherein the substrate polypeptide is a fusion protein, preferably a non-natural fusion protein.
